(19) Europäisches Patentamt
European Patent Office
Office européen des brevets

(11) Veröffentlichungsnummer : **0 100 520**
**B1**

# (12) EUROPÄISCHE PATENTSCHRIFT

(45) Veröffentlichungstag der Patentschrift : **28.05.86**

(51) Int. Cl.[4] : **C 07 D307/60**

(21) Anmeldenummer : **83107367.1**

(22) Anmeldetag : **27.07.83**

## (54) Verfahren zur Herstellung von 4,4-Dimethyl-tetrahydrofuran-2,3-dion.

(30) Priorität : **04.08.82 DE 3229026**

(43) Veröffentlichungstag der Anmeldung : **15.02.84 Patentblatt 84/07**

(45) Bekanntmachung des Hinweises auf die Patenterteilung : **28.05.86 Patentblatt 86/22**

(84) Benannte Vertragsstaaten : **BE CH DE FR GB IT LI NL**

(56) Entgegenhaltungen :
**EP-A- 0 050 721**
**CH-A- 427 770**
**Helv. Chim. Acta, 65, 1982 S. 2024-28**

(73) Patentinhaber : **BASF Aktiengesellschaft**
**Carl-Bosch-Strasse 38**
**D-6700 Ludwigshafen (DE)**

(72) Erfinder : **Halbritter, Klaus, Dr.**
**Schwarzwaldstrasse 19**
**D-6800 Mannheim 1 (DE)**
Erfinder : **Eggersdorfer, Manfred, Dr.**
**Weinbietstrasse 22**
**D-6718 Gruenstadt (DE)**

**EP 0 100 520 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur Herstellung von 4,4-Dimethyl-tetrahydrofuran-2,3-dion (I)

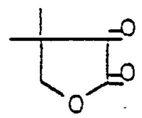

(I)

Diese auch als α-Ketopantolacton oder als 4,4-Dimethyl-dihydrofuran-2,3-dion bezeichnete Verbindung ist ein wichtiges Zwischenprodukt für die Herstellung von D-(—)-Pantolacton (4,4-Dimethyl-2-hydroxybutyrolacton) durch asymmetrische Hydrierung.

Aus der Arbeit von Kuhn und Wieland in Chem. Ber. *75* (1942), Seite 121, ist es bekannt, Dimethylbrenztraubensäure und Formaldehyd in wäßriger Lösung mittels Kaliumcarbonat zu 3,3-Dimethyl-2-oxo-4-hydroxybuttersäure umzusetzen und diese anschließend in salzsaurem Milieu zur Verbindung I zu lactonisieren. Die hierbei erzielte Ausbeute betrug 60 %, jedoch ist es nach Lipton und Strong, J. Am. Chem. Soc. *71*, 1949, S. 2364, nicht sicher, ob es sich bei dem Produkt tatsächlich um I handelt.

Die letztgenannten Autoren haben I daher auf anderem Wege hergestellt, nämlich durch Oxidation von Pantolacton mit Bleitetraacetat, erhielten aber nur 33 % Ausbeute.

Die Verbesserung des Oxidationsverfahrens führte schließlich bei Verwendung von Alkali- oder Erdalkalimetallhypochloriten als Oxidationsmittel zu Ausbeuten von 70-90 % (EP-Al 0 006 156), jedoch ist diese Methode nicht befriedigend, weil es sich um ein heterogenes Reaktionsgemisch handelt, dessen Aufarbeitung verfahrenstechnische Schwierigkeiten bedingt.

Aus der EP-A1-50721 ist es ferner bekannt, Ketopantolacton durch Umsetzung eines Alkali- oder Erdalkalimetallsalzes der Dimethylbrenztraubensäure und Formaldehyd in Gegenwart einer anorganischen Base herzustellen, wobei man Ausbeuten zwischen 70 und 87 % erzielt. Dieses Verfahren ist jedoch verfahrenstechnisch nachteilig, weil man hierbei zunächst die Salze der Dimethylbrenztraubensäure herstellen muß und weil die Handhabung von Feststoffen in der Technik stets umständlich ist. Über die Herstellung der Salze ist in dieser EP-A1 zwar nichts gesagt, jedoch empfiehlt der Erfinder Fizet in seiner nachveröffentlichten Arbeit in Helv. Chim. Acta, *65*, 1982, Seiten 2024-28, hierzu als beste Methode gemäß Schema 4 und den Ausführungen auf Seite 2026 oben die Verseifung des Dimethylbrenztraubensäureethylester, d. h. in der Fachwelt bestand offensichtlich ein Vorurteil gegen den wesentlich billigeren und bequemeren unmittelbaren Einsatz der Ester. Dieses Vorurteil beruht vermutlich darauf, daß die Ester bei den niederen Temperaturen von etwa 20-50 °C sehr langsam im gewünschten Sinne reagieren und daß man bei höheren Temperaturen die erhebliche Zunahme von Nebenreaktionen infolge der stark aktivierten Oxogruppen der Brenztraubensäure befürchtet hat.

Der vorliegenden Erfindung lag daher die Aufgabe zugrunde, die Titelverbindung auf einfachere und wirtschaftlichere Weise zugänglich zu machen.

Demgemäß wurde ein Verfahren zur Herstellung von 4,4-Dimethyltetrahydrofuran-2,3-dion (I) gefunden, welches dadurch gekennzeichnet ist, daß man einen Dimethylbrenztraubensäureester (II) bei 80-150 °C mit Formaldehyd oder einer Formaldehyd abspaltenden Verbindung umsetzt.

Dieses Verfahren ist bemerkenswert, da es entgegen jeglicher Erwartung in glatter Reaktion zu Ausbeuten von 80-90 %, bezogen auf II, führt.

Auf den Alkoholrest im Ausgangsester II kommt es nach den bisherigen Beobachtungen grundsätzlich nicht an, so daß sich II von aliphatischen, cycloaliphatischen, araliphatischen und aromatischen Alkoholen ableiten kann. Aus praktischen Gründen werden jedoch die Ester von Alkanolen mit 1-8, vorzugsweise 1-4 C-Atomen bevorugt, darunter vor allem des Methanols, Ethanols, Isopropanols, Isobutanols und tert.-Butanols.

Mit steigendem Molekulargewicht des Alkohols sinkt zwar die Reaktionsgeschwindigkeit, jedoch fällt dieser Effekt nicht insoweit ins Gewicht, daß das Verfahren dadurch unattraktiv werden würde, zumal man ihn durch Wahl schärferer Reaktionsbedingungen weitgehend kompensieren kann. Man kann daher beispielsweise auch von den Estern des Cyclohexanols, Benzylalkohols und Phenols ausgehen.

Die Dimethylbrenztraubensäureester II sind ihrerseits durch Umsetzung von Isobutyraldehyd mit Blausäure und den entsprechenden Alkoholen sowie durch anschließende oxidative Dehydierung in hohen Ausbeuten zugänglich.

Der Formaldehyd kann in Form von wäßrigen oder organischen Lösungen, gasförmig oder als Formaldehyd abspaltende Substanz, z. B. als Paraformaldehyd, eingesetzt werden. Da der Formaldehyd reduzierende Eigenschaften hat und die 3-Ketogruppe von I bzw. II unerwünschterweise zu hydrieren vermag, setzt man ihn zweckmäßigerweise nicht in größerem Überschuß sondern etwa im Verhältnis von 1.0 : 1 bis 1,3 : 1, zum Ester II ein.

Prinzipiell kann man die Umsetzung lösungsmittelfrei vornehmen, etwa indem man gasförmigen Formaldehyd in den flüssigen Ester II einleitet oder Paraformaldehyd unter Zusatz von etwas Säure mit II reagieren läßt. Aus verfahrenstechnischen Gründen empfiehlt es sich jedoch, die Reaktion in Gegenwart

eines Lösungs- oder Verdünnungsmittels in heterogener oder vorzugsweise homogener Phase vorzunehmen.

Als Lösungs- und Verdünnungsmittel eignen sich beispielsweise Wasser und besonders organische Lösungsmittel wie vor allem $C_6$-$C_{12}$-Kohlenwasserstoffe und $C_1$-$C_4$-Alkanole, Ether wie Diethylether, Ketone wie Aceton, chlorierte Kohlenwasserstoffe oder Gemische dieser Flüssigkeiten. Besonders vorteilhaft ist es, den Formaldehyd in Form seiner Lösungen in den organischen Lösungsmitteln, besonders den $C_1$-$C_4$-Alkanolen, einzusetzen. Im allgemeinen beträgt die Menge des Lösungs- oder Verdünnungsmittels 0,5-20 l pro kg II. Größere Mengen schaden nicht, erhöhen aber naturgemäß den Aufarbeitungsaufwand.

Das Verfahren gelingt bereits ohne die Gegenwart katalytisch wirksamer Substanzen äußerst befriedigend, kann jedoch durch die Mitverwendung saurer oder basischer Katalysatoren begünstigt werden, wodurch sich die Reaktionstemperaturen und/oder die Reaktionszeiten senken lassen.

Als Katalysatoren kommen anorganische Säuren wie Schwefelsäure, Salzsäure oder Natriumhydrogensulfat, organische Säuren wie Ameisensäure oder Essigsäure, anorganische Basen wie Natriumhydroxid, -carbonat, -methylat oder die entsprechenden Kaliumverbindungen und organische Basen wie tertiäre Stickstoffbasen, beispielsweise Trimethyl- und Triethylamin, in Betracht. Die wirksame Menge dieser Katalysatoren liegt im allgemeinen zwischen 0,01 und 1 Gew.% von II. Größere Mengen sind möglich, bringen in der Regel aber kaum noch wirtschaftliche Vorteile.

Unterhalb 80 °C verläuft die Reaktion unwirtschaftlich langsam und oberhalb 150 °C machen sich Nebenreaktionen in verstärktem Ausmaß bemerkbar. Bevorzugt wird der Temperaturbereich von 100-150 °C, vor allem von 130-145 °C. Durch Mitverwendung eines Katalysators kann die Temperatur bei gleichen Reaktionszeiten etwa um 10-20 °C erniedrigt werden. Umgekehrt läßt sich die Reaktionszeit mittels der Katalysatoren bei gleicher Temperatur bis auf etwa die Hälfte senken.

Entsprechend den Reaktionstemperaturen wird die Reaktion unter dem dazugehörigen Eigendruck, der zwischen etwa 1 und 4 bar liegt, ausgeführt.

Im Gegensatz zur Arbeitsweise von Kuhn und Wieland (cit.) verlaufen Formaldehyd-Addition und Ringschluß praktisch gleichzeitig, so daß im Reaktionsgemisch kein 3,3-Dimethyl-2-oxo-4-hydroxybuttersäureester nachweisbar ist. Dies ist von besonderem Vorteil, weil hierdurch ein Verfahrensschritt, nämlich die getrennte Lactonisierung, entfällt.

Die Reaktionszeiten für einen praktisch quantitativen Umsatz von II betragen je nach den Reaktionsbedingungen in der Regel 2-5 Stunden.

Im übrigen kann man das Verfahren nach den üblichen Techniken diskontinuierlich oder kontinuierlich vornehmen. Dies gilt auch für die Aufarbeitung des Reaktionsgemisches, die vorzugsweise destillativ erfolgt. Wird I weiter umgesetzt, so kann sich dessen Isolierung ber auch erübrigen.

## Beispiel 1

In einem Rührautoklaven von 250 ml Inhalt wurden 68,8 g (0,4 mol) Dimethylbrenztraubensäureisobutyl-ester und eine Lösung aus 24 g Isobutanol und 12 g Formaldehyd (= 0,4 mol $CH_2O$) 2 Stunden lang auf 110 °C erhitzt. Die übliche destillative Aufarbeitung des Reaktionsgemisches lieferte das reine α-Ketopantolacton in 89 %iger Ausbeute Sdp. 85-90 °C/2 mbar, Fp. 68-70 °C.

## Beispiel 2

Die Umsetzung von 58 g (0,4 mol) Dimethylbrenztraubensäureethylester und 44 g 30 gew.%iger wäßriger Formaldehydlösung (= 0,44 mol $CH_2O$) bei 145 °C und einer Reaktionszeit von 3 Stunden sowie anschließender Extraktion von I mittels Diethylether und Destillation der Extraktphase lieferte das reine Verfahrensprodukt in 83 %iger Ausbeute.

## Beispiel 3

Analog Beispiel 2, jedoch unter zusätzlicher Verwendung von 0,4 g Triethylamin als Katalysator, fiel das Verfahrensprodukt in 87 %iger Ausbeute an. Die Reaktionstemperatur betrug in diesem Falle 135 °C und die Reaktionszeit 2,5 Stunden.

## Beispiel 4

Analog Beispiel 2, jedoch unter zusätzlicher Verwendung von 0,3 g Schwefelsäure als Katalysator, wurde das Verfahrensprodukt in einer Ausbeute von 83 % erhalten. Die Reaktionstemperatur betrug in diesem Falle 140 °C und die Reaktionsdauer 2 Stunden.

**Patentanspruch**

Verfahren zur Herstellung von 4,4-Dimethyltetrahydrofuran-2,3-dion (I), dadurch gekennzeichnet,

daß man einen Dimethylbrenztraubensäureester (II) bei 80-150 °C mit Formaldehyd oder einer Formaldehyd abspaltenden Verbindung umsetzt.

**Claim**

A process for the preparation of 4,4-dimethyltetrahydrofuran-2,3-dione (I), wherein a dimethylpyruvate (II) is reacted with formaldehyde or a formaldehyde donor at 80-150 °C.

**Revendication**

Procédé de préparation de la diméthyl-4,4 tétrahydrofuranne dione-2,3 (I), caractérisé en ce que l'on fait réagir un ester de l'acide diméthyl pyruvique (II) entre 80 et 150 °C avec le formaldéhyde ou avec un composé libérant du formaldéhyde.